# NEUE EUROPÄISCHE PATENTSCHRIFT

(11) **EP 0 378 785 B2**
(45) Veröffentlichungstag und Bekanntmachung des Hinweises auf die Entscheidung über den Einspruch: **08.01.1997**
(45) Hinweis auf die Patenterteilung: 07.04.1993
(21) Anmeldenummer: 89121617.8
(22) Anmeldetag: 23.11.1989
(51) Int. Cl.: C09C 3/12, C08K 9/06, C08L 83/04

(54) **Verfahren zur Hydrophobierung von Si-OH-Gruppen enthaltenden Feststoffteilchen und Verwendung derselben**
Process for hydrophobing solid particles containing Si-OH groups, and their use
Procédé pour rendre hydrophobes les particules solides contenant des groupes Si-OH et leur utilisation

(30) Priorität: 25.11.1988 DE 3839900
(43) Veröffentlichungstag der Anmeldung: 25.07.1990
(73) Patentinhaber: WACKER-CHEMIE GMBH, D-81737 München (DE)
(72) Erfinder: Schuster, Johann, Dr., D-8261 Emmerting (DE); Müller, Horst, Dr., D-8261 Emmerting (DE); Vorbuchner, Helmut, D-8263 Burghausen (DE); Maier, Anton, D-8263 Burghausen (DE); Pradl, Ferdinand, Dr., D-8263 Burghausen (DE)

(56) Entgegenhaltungen:
- EP-A- 0 326 810
- DE-A- 2 403 783
- DE-A- 2 535 334
- DE-A- 2 728 490
- FR-A- 1 157 863
- GB-A- 783 868
- GB-A- 2 056 995
- US-A- 3 024 126
- US-A- 4 780 108

## Beschreibung

Die Erfindung betrifft ein Verfahren zur Herstellung von hydrophobem, teilchenförmigem Siliciumdioxyd.

In der DE-OS 23 44 388 bzw. der entsprechenden US 3953487 wird erwähnt, daß die Hydrophobierung von Siliciumdioxyd in inerten, organischen Lösungsmitteln und einem hochtourigen, mit über 2000 Umdrehungen pro Minute betriebenen Homogenisier- und Dispergiergerät durchgeführt werden kann. Bei den angegebenen hohen Drehzahlen erfolgt jedoch ein hoher Materialverschleiß und Abrieb. Weiter werden bei dieser Hydrophobierung 3 bis 25 Gew.-% an Hydrophobierungsmittel, bezogen auf den Feststoff, verwendet, was lange Reaktionszeiten bedingt, woraus erhöhter Energieaufwand und schlechte Raum-Zeit-Ausbeuten resultieren.

Bei der Herstellung von zu Elastomeren härtbaren Massen auf Basis von Diorganopolysiloxanen unter Verwendung von hydrophobem Füllstoff geht man im Stand der Technik, wie beispielsweise in der DE-A-25 35 334 offenbart, zumeist derart vor, daß die Hydrophobierung beim Mischen des Füllstoffes mit dem Diorganopolysiloxan durch Zugabe eines Hydrophobierungsmittels, also in-situ, stattfindet. Die Verwendung von hydrophobem Füllstoff, der im Wirbelbett, Kollergang, Rührwerkskugelmühle etc. hydrophobiert wurde, wie beispielsweise in der DE-OS 22 11 377 bzw. der entsprechenden US 3868345 offenbart, in zu Elastomeren härtbaren Massen ist in vielen Fällen nicht möglich, da sich die Eigenschaften der daraus hergestellten Produkte von den nach dem in-situ-Verfahren erzeugten Produkten unterscheiden. Das in-situ-Verfahren weist jedoch die Nachteile langer Chargen laufzeit und hoher Emissionen, die an vielen Stellen auftreten und dadurch schwer beherrschbar werden, auf. Weiter ist eine gezielte Steuerung der Hydrophobierung kaum möglich und auch Korrekturen der Füllstoffgehalte der zu Elastomeren härtbaren Massen auf Basis von Diorganopolysiloxanen sind nicht mehr möglich, da geeignete Füllstoffe fehlen.

Der Gegenstand der Erfindung ist ein Verfahren zur Herstellung von hydrophobem, teilchenförmigem Siliciumdioxyd durch Umsetzung eines Hydrophobierungsmittels auf Basis von Organosiliciumverbindungen mit Si-OH-Gruppen enthaltendem, teilchenförmigem, pyrogen erzeugtem oder gefälltem Siliciumdioxyd unter gleichzeitiger mechanischer Beanspruchung des Reaktionsgemisches, das dadurch gekennzeichnet ist, daß 5 bis 50 Gew.-% des Si-OH-Gruppen enthaltenden, teilchenförmigen, pyrogen erzeugten oder gefällten Siliciumdioxyds, bezogen auf das Gesamtgewicht der Reaktionsmischung, bestehend aus teilchenförmigem, pyrogen erzeugtem oder gefälltem Siliciumdioxyd und Hydrophobierungsmittel, wobei das Hydrophobierungsmittel 1 bis 5 Gew.-% Wasser bezogen auf das Gesamtgewicht des Hydrophobierungsmittels enthält, eingesetzt werden, mit der Maßgabe, daß das Reaktionsgemisch kein lineares Polyorganosiloxan der allgemeinen Formel 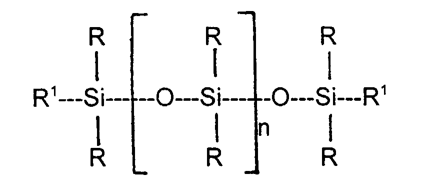 wobei R gleiche oder verschiedene einwertige substituierte oder nichtsubstituierte Kohlenwasserstoffreste darstellt, R¹ dieselbe Bedeutung wie R hat und darüber hinausgehend einen OH-Rest darstellen kann und n eine ganze positive Zahl ist, enthält.

Durch die separate Hydrophobierung des teilchenförmigen, pyrogen erzeugten oder gefällten Siliciumdioxyds ist es möglich, den Hydrophobierungsgrad gezielt zu steuern und in weiten Grenzen zu variieren, wobei hohe und/oder gleichmäßige Hydrophobierungsgrade möglich sind, was für viele Anwendungen Voraussetzung ist. Dies sollte vor allem erreicht werden, ohne daß sauere bzw. alkalische Rückstände verbleiben. Auch neutrale Salze oder andere Zusätze, bei denen es sich nicht um siliciumorganische Verbindungen handelt, sollten nicht im Feststoff verbleiben. Das erfindungsgemäße Verfahren erlaubt es, teilchenförmiges, pyrogen erzeugtes oder gefälltes Siliciumdioxyd so zu hydrophobieren, daß damit zu Elastomeren härtbare Massen auf Basis von Diorganopolysiloxanen, die Feststoffe enthalten und im Stand der Technik nur durch in-situ-Verfahren herstellbar sind, durch einfaches Mischen des hydrophobierten, teilchenchenförmigen Siliciumdioxyds mit Diorganopolysiloxan erzeugt werden können. Die Verwendung von vorab hydropobiertem, teilchenförmigem Siliciumdioxyd führt zu einer deutlichen Kapazitätssteigerung der Mischorgane. Emissionen werden auf eine zentrale Anlage begrenzt und sind dadurch leichter beherrschbar. Der Verbrauch an Hydrophobierungsmittel kann gegenüber dem in-situ-Verfahren deutlich reduziert werden. Der Feststoffgehalt der Massen läßt sich nachträglich leicht durch Zusatz von weiterem, erfindungsgemäßem Siliciumdioxyd korrigieren. Für die Herstellung von Flüssigkautschuk, kerbfesten additions- und kondensationsvernetzenden Ein- und Zweikomponentensiliconkautschukmassen eignet sich nur erfindungsgemäß hergestelltes Siliciumdioxyd. Nur bei dessen Verwendung kann je nach Produkt gutes Fließverhalten, gute Transparenz und geringe Flüchtigkeit erreicht werden.

Das erfindungsgemäße Verfahren wird unter gleichzeitiger mechanischer Beanspruchung des Reaktionsgemisches vorzugsweise in einem Mischorgan bei Drehzahlen von vorzugsweise 300 bis 2000 Umdrehungen pro Minute, insbesondere 300 bis 1500 Umdrehungen pro Minute, durchgeführt.

Beispiele für Mischorgane sind Turrax, Dissolver, Henschelmischer und Mischturbine.

Das erfindungsgemäße Verfahren wird vorzugsweise in inerter Atmosphäre durchgeführt, wobei der Gehalt an Sauerstoff auf zumindest 3 Vol.-% reduziert ist. Vorzugsweise wird in Stickstoff- oder Argonatmosphäre gearbeitet.

Nach beendeter Hydrophobierung wird das überschüssige Hydrophobierungsmittel entfernt und vorzugsweise beim nächsten Ansatz erneut eingesetzt. Abreagiertes Hydrophobierungsmittel sowie Verluste werden ersetzt.

Durch Variation der Drehzahl des Mischorgans oder der Verweilzeit läßt sich der Hydrophobierungsgrad des erhaltenen, hydrophoben, teilchenförmigen Siliciumdioxyds leicht variieren. Bevorzugte Verweilzeiten sind 10 bis 1800 Sekunden.

Das Verfahren läßt sich sowohl kontinuierlich als auch diskontinuierlich durchführen.

Es werden 5 bis 50 Gew.-%, vorzugsweise 20 bis 30 Gew.-%, des Si-OH-Gruppen enthaltenden teilchenförmigen, pyrogen erzeugten oder gefällten Siliciumdioxyds, bezogen auf das Gesamtgewicht der Reaktionsmischung, bestehend aus teilchenförmigem, pyrogen erzeugten oder gefällten Siliciumdioxyd, und Hydrophobierungsmittel, eingesetzt. Die Mengenverhältnisse sind beim erfindungsgemäßen Verfahren jedoch immer derart gestaltet, daß das Reaktionsgemisch, bestehend aus teilchenförmigem, pyrogen erzeugtem oder gefälltem Siliciumdioxyd und Hydrophobierungsmittel, eine pastöse Konsistenz aufweist.
Durch diese pastöse Konsistenz gelingt es, das Reaktionsgemisch auch bei geringen Drehzahlen des Mischorgans hohen Scherkräften auszusetzen.
Diese hohen Scherkräfte führen zu hoher mechanischer Beanspruchung des Reaktionsgemisches, wodurch Agglomerate des teilchenförmigen Feststoffes zerkleinert werden, was wiederum eine Erhöhung der Hydrophobierung bedingt.

Das Si-OH-Gruppen enthaltende, teilchenförmige Siliciumdioxyd weist vorzugsweise eine Oberfläche von 5 m²/g bis 600 m²/g, insbesondere 150 m²/g bis 300 m²/g, nach BET auf.

Als Hydrophobierungsmittel auf Basis von Organosiliciumverbindungen können im Rahmen der Erfindung die gleichen verwendet werden, die bisher zur Hydrophobierung von Si-OH-Gruppen enthaltendem, teilchenförmigem Siliciumdioxyd verwendet wurden. Diese Hydrophobierungsmittel enthalten 1 bis 5 Gew.-% Wasser, bezogen auf das Gesamtgewicht des Hydrophobierungsmittels.

Zusammen mit Wasser können, falls gewünscht, an sich bekannte, die Umsetzung von Si-OH-Gruppen enthaltendem, feinteiligem Siliciumdioxyd mit Organosiliciumverbindungen fördernde Katalysatoren, wie Chlorwasserstoff, Amine, z.B. n-Butylamin und/oder Metallverbindungen, z.B. Titantetrachlorid oder Dibutylzinndilaurat, mitverwendet werden.

Bevorzugte Organosiliciumverbindungenfür Hydrophobierungsmittel sind solche der allgemeinen Formel

(R₃Si)ₐZ,

worin R gleiche oder verschiedene, einwertige, gegebenenfalls substituierte Kohlenwasserstoffreste bedeutet, Z Halogen, Wasserstoff oder ein Rest der Formel -OH, -OR',-NR'X, -ONR'₂, -OOCR', -O- oder -N(X)-, wobei R' meist ein Alkylrest mit 1 bis 4 Kohlenstoffatomen ist und X Wasserstoff ist oder die gleiche Bedeutung wie R' hat, und a 1 oder 2 ist. Das bei weitem wichtigste Beispiel für einen Kohlenwasserstoffrest R ist der Methylrest. Weitere Beispiele für Kohlenwasserstoffreste R sind Octadecylreste, der Phenyl- sowie der Vinylrest.

Beispiele für substituierte Kohlenwasserstoffreste R sind insbesondere halogenierte Kohlenwasserstoffreste wie der 3,3,3-Trifluorpropylrest.

Beispiele für Reste R' sind der Methyl-, Ethyl- und Propylrest.

Beispiele für Organosiliciumverbindungen der oben angegebenen Formel sind Hexamethyldisilazan, Trimethylsilanol, Trimethylchlorsilan, Trimethylethoxysilan, Triorganosilyloxyacylate, wie Vinyldimethylacetoxysilan, Triorganosilylamine, wie Trimethylsilylisopropylamin, Trimethylsilylethylamin, Dimethylphenylsilylpropylamin und Vinyldimethylsilylbutylamin, Triorganosilylaminooxyverbindungen, wie Diethylaminooxytrimethylsilan und Diethylaminooxydimethylphenylsilan, ferner Hexamethyldisiloxan, 1,3-Divinyltetramethyldisiloxan, 1,3-Diphenyltetramethyldisiloxan und 1,3-Diphenyltetramethyldisilazan. Weitere Beispiele für Organosiliciumverbindungen sind Dimethyldichlorsilan, Dimethyldiethoxysilan, Dimethyldimethoxysilan, Diphenyldiethoxysilan, Vinylmethyldimethoxysilan, Methyltriethoxysilan, Octamethylcyclotetrasiloxan.

Es können auch Gemische aus verschiedenen Organosiliciumverbindungen mit dem teilchenförmigen, Si-OH-Gruppen enthaltenden, pyrogen erzeugten oder gefällten Siliciumdioxyd umgesetzt werden.

Besonders gute Ergebnisse werden erreicht, wenn man Hydrophobierungsmittel verwendet, die aus
70 bis 89 Gew.-% Hexamethyldisiloxan und/oder Trimethylsilanol,
10 bis 30 Gew.-% Hexamethyldisilazan und/oder Divinyltetramethyldisilazan und 1 bis 5 Gew.-% Wasser bestehen.
Die Angaben in Gew.-% beziehen sich hierbei auf das Gesamtgewicht des Hydrophobierungsmittels.

Die genannten Mischorgane sind meist weder mit Einrichtungen zum Heizen noch mit Einrichtungen zum Erstellen des Drucks, der von der umgebenden Atmosphäre abweicht, ausgestattet. Die Hydrophobierung wird daher vorzugsweise ohne zusätzliche Erwärmung und beim Druck der umgebenden Atmosphäre, also 1080 hPa(abs.) oder ungefähr 1080 hPa(abs.), durchgeführt. Falls möglich und erwünscht können jedoch auch andere Temperaturen bis zum Siedepunkt des Hydrophobierungsmittels und/oder andere Drücke im Bereich von vorzugsweise 1000 bis 10000 hPa(abs.) bei der Hydrophobierung angewandt werden.

Das durch das erfindungsgemäße Verfahren erhaltene, hydrophobe, teilchenförmige Siliciumdioxyd fällt ohne zusätzlichen Verdichtungsschritt mit hohem Schüttgewicht an, was für die Weiterverarbeitung von Vorteil ist. Das gegenüber dem Ausgangsmaterial höhere Schüttgewicht resultiert aus der Zerstörung von voluminösen Agglomeraten.

Bei der kontinuierlichen Durchführung des Verfahrens zur Herstellung von hydrophobem, teilchenförmigem Siliciumdioxyd hat sich die in der Figur dargestellte Ausführungsform besonders bewährt. Hierin bedeuten:
1 Mischbehälter
2 Pumpe
3 Mischzelle mit Mischorgan
4 Trockner-Anlage
5 Kondensator
6 Pufferbehälter
7 beheizter Staubfilter

Das erfindungsgemäß hergestellte Siliciumdioxyd findet Verwendung in zu Elastomeren härtbaren Massen.
Als Diorganopolysiloxane können beim Verfahren zur Herstellung von zu Elastomeren härtbaren Massen auf Basis von Diorganopolysiloxanen, die Siliciumdioxyd enthalten, alle Diorganopolysiloxane verwendet werden, die bisher als Grundlage für bei Raumtemperatur oder erhöhter Temperatur härtbare bzw. härtende Massen verwendet wurden bzw. verwendet werden können. Sie können z.B. durch die allgemeine Formel

Z¹ ₙSi(R¹)O₃₋ₙ[Si(R¹ ₂)O]ₓSi(R¹)₃₋ₙZ¹ ₙ

wiedergegeben werden, worin R¹ gleiche oder verschiedene, einwertige gegebenenfalls substituierte und/oder polymere Kohlenwasserstoffreste, und Z¹ eine Hydroxylgruppe, hydrolysierbare Gruppe und/oder hydrolysierbares Atom bedeuten, oder im Fall von Massen, deren Härtung bei erhöhter Temperatur durch Peroxide initiiert wird, kann Z¹ auch einen Alkylrest darstellen, n kann die Werte 1, 2 oder 3 annehmen und x bedeutet eine ganze Zahl von mindestens 1.

Innerhalb bzw. entlang der Siloxankette in der oben angegebenen Formel können, was in derartigen Formeln üblicherweise nicht dargestellt wird, auch andere meist nur als Verunreinigungen vorliegende Siloxaneinheiten als Diorganosiloxaneinheiten, z.B. solche der Formeln

R¹SiO_{3/2}, R¹ ₃SiO_{1/2} und SiO_{4/2},

wobei R¹ jeweils die oben dafür angegebene Bedeutung hat, vorhanden sein. Die Menge dieser anderen Siloxaneinheiten sollte 10 Molprozent nicht überschreiten.

Neben den kettenförmigen Siloxanmolekülen können die verwendeten
Diorganopolysiloxane auch bis zu 20 Gew.-% cyclisch aufgebaute Siloxaneinheiten der Formel

(R¹ ₂Si-O)ₓ,

wobei R¹ und x jeweils die oben dafür angegebene Bedeutung haben, enthalten.

Beispiele für Kohlenwasserstoffreste R¹ sind Alkylreste, wie Methyl-, Ethyl-, Propyl-, Butyl-, Hexyl- und Octylreste; Alkenylreste, wie der Vinyl-, Allyl-, Ethylallyl- und Butadienylrest; und Arylreste, wie der Phenyl- und Tolylrest.

Beispiele für substituierte Kohlenwasserstoffreste R¹ sind insbesondere halogenierte Kohlenwasserstoffreste wie der 3,3,3-Trifluorpropylrest, Chlorphenyl- und Bromtolylreste; und Cyanalkylreste, wie der beta-Cyanethylrest.

Beispiele für polymere (auch als "modifizierende" bezeichenbare) substituierte und unsubstituierte Kohlenwasserstoffreste R¹ sind über Kohlenstoff an Silicium gebundene Polystyryl-, Polyvinylacetat-, Polyacrylat-, Polymethacrylat- und Polyacrylnitrilreste.

Mindestens der überwiegende Teil der Reste R¹ besteht vor allem wegen der leichten Zugänglichkeit vorzugsweise aus Methylgruppen. Die gegebenenfalls vorhandenen übrigen Reste R¹ sind insbesondere Vinyl- und/oder Phenylgruppen.

Insbesondere im Fall des Vorliegens von unter Ausschluß von Wasser lagerfähigen, bei Zutritt von Wasser bei Raumtemperatur zu Elastomeren härtenden Massen handelt es sich bei Z¹ meist um hydrolysierbare Gruppen. Beispiele für solche Gruppen sind Amino-, Amido-, Aminoxy-, Oxim-, Alkoxy-, Alkoxy-alkoxy- (z.B. CH₃OCH₂CH₂O-), Alkenyloxy- (z.B. H₂C=(CH₃)CO-), Acyloxy- und Phosphatgruppen. Vor allem wegen der leichten Zugänglichkeit sind als Z¹ Acyloxygruppen, insbesondere Acetoxygruppen bevorzugt. Es werden jedoch auch z.B. mit Oximgruppen, wie solchen der Formel ON=C(CH₃)(C₂H₅), als Z¹ ausgezeichnete Ergebnisse erzielt.

Beispiele für hydrolysierbare Atome Z¹ sind Halogen- und Wasserstoffatome.

Beipiele für Alkenylgruppen sind insbesondere Vinylgruppen.

Die Viskosität der verwendeten Diorganopolysiloxane beträgt je nach Endprodukt vorzugsweise zwischen 20 mPas und 50 000 000 mPas (25 °C). Dementsprechend beträgt der Wert für x vorzugsweise 15 bis 5000.

Es können auch Gemische aus verschiedenen Diorganopolysiloxanen eingesetzt werden.

Aus den erfindungsgemäß hergestellten, hydrophoben, teilchenförmigen Siliciumdioxyd werden durch Vermischen mit Diorganopolysiloxanen und gegebenenfalls weiteren Stoffen bei Raumtemperatur oder nur wenig erhöhter Temperatur, gegebenenfalls nach Zusatz von Vernetzungsmitteln, zu Elastomeren härtbare Massen hergestellt. Dieses Vermischen kann in beliebiger bekannter Weise, z.B. in mechanischen Mischgeräten, erfolgen.

Vorzugsweise werden zumindest 10 Gew.-%, insbesondere 30 - 100 Gew.-%, bezogen auf das Gesamtgewicht an eingesetztem Füllstoff, erfindungsgemäß hergestellte, hydrophobe, teilchenförmige Füllstoffe verwendet.

Vorzugsweise werden die Füllstoffe in Mengen von mindestens 5 Gewichtsprozent, insbesondere 5 bis 50 Gew.-%, bezogen auf das Gesamtgewicht der zu Elastomeren härtbaren Massen, eingesetzt.

Liegen in den reaktionsfähige endständige Einheiten enthaltenden Diorganopolysiloxanen als einzige reaktionsfähige endständige Einheiten solche mit Si-gebundenen Hydroxylgruppen vor, so müssen diese Diorganopolysiloxane, um sie in an sich bekannter Weise zu härten bzw. um sie in durch das in der Luft enthaltene Wasser, gegebenenfalls unter Zugabe von weiterem Wasser, zu Elastomeren härtende Verbindungen zu überführen, mit Vernetzungsmitteln, gegebenenfalls in Gegenwart eines Kondensationskatalysators in bekannter Weise umgesetzt werden.

Beispiele für solche Vernetzungsmittel sind insbesondere Silane der allgemeinen Formel

R¹ ₄₋ₜSiZ¹'ₜ,

worin R¹ die oben dafür angegebene Bedeutung hat, Z¹' eine hydrolysierbare Gruppe und/oder ein hydrolysierbares Atom und t 3 oder 4 ist. Die für Z¹ oben angeführten Gruppen und Atome gelten im vollen Umfang auch für die hydrolysierbaren Gruppen Z¹' und die hydrolysierbaren Atome Z¹'.

Beispiele für Silane der oben angegebenen Formel sind Methyltriacetoxysilan, Isopropyltriacetoxysilan, Isopropoxytriacetoxysilan, Isopropoxytriacetoxysilan, Vinyltriacetoxysilan, Methyltrisdiethylaminoxysilan, Methyltris (cyclohexylamino)-silan, Methyltris(-diethylphosphato)-silan und Methyltris(-methyl-ethylketoximo)-silan.

Anstelle von oder im Gemisch mit Silanen der oben angegebenen Formel können ferner z.B. auch Polysiloxane verwendet werden, die je Molekül mindestens 3 Z¹'-Gruppen bzw. -Atome, enthalten, wobei die nicht durch Z¹'-Gruppen bzw. -Atome abgesättigten Siliciumvalenzen durch Siloxansauerstoffatome und gegebenenfalls R¹-Gruppen abgesättigt sind. Die bekanntesten Beispiele für Vernetzer der letzteren Art sind das Polyethylsilikat mit einem SiO₂-Gehalt von etwa 40 Gewichtsprozent, Hexamethoxydisiloxan und Methylwasserstoffpolysiloxane.

Die bekanntesten Beispiele für Kondensationskatalysatoren sind Zinnsalze von Fettsäuren, wie Dibutylzinndilaurat, Dibutylzinndiacetat und Zinn-(ll)-octoat.

Liegen in den reaktionsfähige endständige Einheiten enthaltenden Diorganopolysiloxanen als einzige reaktionsfähige, endständige Einheiten solche mit Alkenylgruppen vor, so kann die Härtung zum Elastomeren in bekannter Weise mit Organopolysiloxanen, die durchschnittlich mindestens 3 Si-gebundene Wasserstoffatome je Molekül enthalten, wie Methylwasserstoffpolysiloxan, in Gegenwart von die Anlagerung von Alkenylgruppen an Si-gebundenen Wasserstoff fördernden Katalysatoren, wie Hexachloroplatinsäure oder Pt-Komplexen, erfolgen.

Schließlich sei als weiteres Beispiel für die Härtung zu Elastomeren diejenige mittels Peroxiden erwähnt. Die Peroxide bewirken dabei eine bei erhöhter Temperatur einsetzende radikalische Vernetzung von Alkyl- und Alkenylgruppen.
Als Peroxide werden z.B. Dibenzoyl-, Dicumyl-, m-Cl-Benzoyl- oder 2,4-Dichlorbenzoylperoxid verwendet.

Selbstverständlich können die zu Elastomeren härtbaren Massen außer Diorganopolysiloxanen, dungsgemäß hergestelltem Siliciumdioxyd, Vernetzungsmitteln und Vernetzungskatalysatoren, gegebenenfalls weitere herkömmlicherweise verwendete Stoffe enthalten. Beispiele für solche Stoffe sind nicht hydrophobierte Füllstoffe mit einer Oberfläche unterhalb 50 m²/g, wie Quarzmehl, Diatomeenerde, sogenannte Molekularsiebe, wie Natriumcalciumaluminiumsilikat, ferner Zirkoniumsilikat und Calciumcarbonat, ferner nicht hydrophobiertes, pyrogen erzeugtes Siliciumdioxyd, organische Harze, wie Polyvinylchloridpulver, Organopolysiloxanharze, faserige Füllstoffe, wie Asbest, Glasfasern, Kohlefasern und organische Fasern, Pigmente, lösliche Farbstoffe, Duftstoffe, Korrosionsinhibitoren, die Massen gegen den Einfluß von Wasser stabilisierende Mittel, wie Essigsäureanhydrid, die Härtung verzögernde Mittel, wie Ethinylcyclohexanol und Weichmacher, wie durch Trimethylsiloxygruppen endblockierte Dimethylpolysiloxane.

### Beispiel 1

In einer 5 l Rührapparatur wurden 1,3 l eines Gemisches aus 60 Gew.-% Trimethylsilanol und 40 Gew.-% Hexamethyldisiloxan vorgelegt, die Apparatur mit Stickstoff inertisiert und dann bei 300 Upm (Umdrehungen pro Minute) 450 g pyrogenes Siliciumdioxyd mit einer Oberfläche von 300 m²/g eingerührt. Anschließend wurden 64 g Hexamethyldisilazan und 7 g Wasser zugegeben. Unter leichter Stickstoffspülung wurde die Paste mittels Dissolver bei 1000 Upm 1 h gemischt. Die Temperatur stieg dabei auf 70 °C an. Anschließend wurden die flüchtigen Bestandteile zuerst bei Normaldruck, dann unter Vakuum abdestilliert und das Siliciumdioxyd bis zur Gewichtskonstanz bei 200 °C getrocknet. Die anschließende Analyse ergab einen Kohlenstoffgehalt von 4,7 Gew.-%.

### Beispiel 2

In einem geschlossenen 751 Dissolver mit Abstreifer wurden 5 kg hochdisperse Kieselsäure mit einer BET-Oberfläche von 300 m²/g sowie 30 kg eines Gemisches, bestehend aus 60 Gew.-% Trimethylsilanol sowie 40 Gew.-% Hexamethyldisiloxan vorgelegt. Nach der Inertisierung der Anlage mit Stickstoff wurden unter Rühren (200 Upm) 2,3 kg Hexamethyldisilazan sowie 0,8 kg Wasser zugegeben. Mittels Membranpumpe wurden dann noch 7 kg der genannten Kieselsäure zudosiert. Die dabei entstehende Paste wurde dann bei 800 Upm 10 min gemischt. Anschließend wurde das überschüssige Hydrophobierungsmittel abdestilliert. Der so erhaltene Feststoff wies einen C-Gehalt von 4,8 % auf.

### Beispiel 3

In einem Mischbehälter (1) wurden 50 kg/h hochdisperse Kieselsäure mit einer BET-Oberfläche von 300 m²/g sowie 170 kg/h eines Hydrophobiermittelgemisches, bestehend aus 154 kg eines Gemisches aus 60 Gew.% Trimethylsilanol, 40 Gew.% Hexamethyldisiloxan sowie 12 kg Hexamethyldisilazan und 4.0 kg Wasser vorgelegt. Der Mischbehälter (1) wurde dabei mit Stickstoff inertisiert Die sich im Mischbehälter bildende Paste wurde mittels einer Pumpe (2) durch eine Mischzelle (3) gepumpt, in der die Paste durch ein mit 800 Upm laufendes Mischorgan stark geschert wurde. Die Mischzelle wurde dabei von unten nach oben durchströmt. Der Überlauf mündete in eine Trockner-Anlage (4), in der das überschüssige Hydrophobierungsmittel vom Feststoff mittels Verdampfung getrennt wurde. Die Trockner-Anlage wurde ebenfalls mit Stickstoff inertisiert. Das überschüssige verdampfte Hydrophobierungsmittel wurde über einen beheizten Staubfilter (7) und einen Kondensator (5) in einen Pufferbehälter (6) zur Zwischenlagerung überführt.

### Beispiel 4

In einem geschlossenen 75 1 Dissolver mit Abstreifer wurden 20 kg Quarzmehl (Sicron 3000, Fa. Quarzwerke Frechen), sowie 30 kg eines Gemisches, bestehend aus 60 Gew.-% Trimethylsilanol sowie 40 Gew.-% Hexamethyldisiloxan vorgelegt, die Anlage mit Stickstoff inertisiert, dann wurden 2,3 kg Hexamethyldisilazan und 0,8 l Wasser zugegeben. Das Gemisch wurde bei 800 Upm 5 min gerührt. Nach dem Abdestillieren des überschüssigen Hydrophobierungsmittels wurde ein hydrophobiertes Quarzmehl erhalten.

### Beispiel 5 und Vergleichsbeispiel

### Herstellung einer Grundmasse für additionsvernetzende Massen

In einem 5 l Laborkneter wurden 500 g eines mit Vinylgruppen terminierten Dimethylpolysiloxans mit einer Viskosität von 20000 mPas (25°C) vorgelegt, auf 150 °C aufgeheizt und mit 390 g eines Füllstoffes versetzt.
Es entstand eine sehr steife Masse, die anschließend mit 410 g des oben genannten Dimethylpolysiloxans verdünnt wurde. Durch Kneten unter Vakuum (10 mbar) bei 150 °C wurden während einer Stunde flüchtige Bestandteile entfernt.
Aus dieser Grundmasse wurden anschließend in einem Planetenmischer eine A- sowie eine B-Komponente hergestellt. Die A-Komponente, die 30 min bei Raumtemperatur und Normaldruck gemischt wurde, enthielt die Grundmasse sowie 100 ppm Hexachloroplatinsäure.
Die B-Komponente, die ebenfalls 30 min bei Raumtemperatur und Normaldruck gemischt wurde, bestand aus 95 Gew.-% Grundmasse sowie 4 Gew.-% eines Siloxanvernetzers mit 0,18 Mol-% Si-H und 1 Gew.-% Divinyltetramethyldisiloxan.

Die Komponenten A und B wurden in einem Verhältnis von 1:1 vermischt und bei Temperaturen von über 100 °C vulkanisiert. Man erhielt Vulkanisate mit folgenden Eigenschaften:

| Beispiel | Füllstoff | Viskosität der B-Komponente (Pa·s) | Hitzetest (%) | Transparenz |
|---|---|---|---|---|
| 5 | gemäß Beispiel 2 | 900 | 11 | sehr gut |
| Vergleich | gemäß DE-OS 2211377 | 3500 | 113 | schlecht |
| Sollwerte gemäß Produktspezifikation | | 700-1000 | <50 | sehr gut |

### Beispiel 6

### Herstellung einer Grundmasse für kondensationsvernetzende Massen

In einem 10 l Laborkneter wurden 2400 g eines OH-Gruppenterminierten Dimethylpolysiloxans mit einer Viskosität von 6000 mPa·s (25°C) vorgelegt und mit 2400 g eines Füllstoffes gemäß Beispiel 1 versetzt. Nach Beendigung der Füllstoffzugabe wurde 1 Stunde geknetet. Anschließend wurde die Masse 3 Stunden bei 150 °C unter Vakuum ausgeheizt und nachfolgend mit 600 g des oben genannten Dimethylpolysiloxans sowie 1200 g eines mit Trimethylsilylgruppen endblockierten Dimethylpolysiloxans mit einer Viskosität von 100 mPa·s (25°C) verdünnt.

### Beispiel 7

### Herstellung einer Grundmasse für Dentalmassen

In einem 5 l Labor kneter wurden 490 g eines mit Vinylgruppen terminierten Dimethylpolysiloxans mit einer Viskosität von 20000 mPa·s (25°C) vorgelegt und mit insgesamt 920 g Füllstoff gemäß Beispiel 4 versetzt. Nach einstündigem Kneten wurde die Masse mit 125 g des oben genannten Dimethylpolysiloxans verdünnt. Aus dieser Grundmasse ließen sich Dentalmassen mit guter Lagerstabilität herstellen.

## Patentansprüche

1. Verfahren zur Herstellung von hydrophobem, teilchenförmigem Siliciumdioxyd durch Umsetzung eines Hydrophobierungsmittels auf Basis von Organosiliciumverbindungen mit Si-OH-Gruppen enthaltendem, teilchenförmigem, pyrogen erzeugtem oder gefälltem Siliciumdioxyd unter gleichzeitiger mechanischer Beanspruchung des Reaktionsgemisches, dadurch gekennzeichnet, daß 5 bis 50 Gew.-% des Si-OH-Gruppen enthaltenden, teilchenförmigen, pyrogen erzeugten oder gefällten Siliciumdioxid, bezogen auf das Gesamtgewicht der Reaktionsmischung, bestehend aus teilchenförmigem Siliciumdioxyd und Hydrophobierungsmittel, wobei das Hydrophobierungsmittel 1 bis 5 Gew.-% Wasser bezogen auf das Gesamtgewicht des Hydrophobierungsmittels enthält, eingesetzt werden, mit der Maßgabe, daß das Reaktionsgemisch kein lineares Polyorganosiloxan der allgemeinen Formel 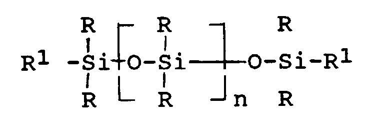 wobei R gleiche oder verschiedene einwertige substituierte oder nichtsubstituierte Kohlenwasserstoffreste darstellt, R¹ dieselbe Bedeutung wie R hat und darüber hinausgehend einen OH-Rest darstellen kann und n eine ganze positive Zahl ist,
enthält.

2. Verfahren nach Anspruch 1, dadurch gekennzeichnet, daß 20 bis 30 Gew.-% des Si-OH-Gruppen enthaltenden, teilchenförmigen, pyrogen erzeugten oder gefällten Siliciumdioxyds, bezogen auf das Gesamtgewicht der Reaktionsmischung, bestehend aus teilchenförmigem Siliciumdioxyd und Hydrophobierungsmittel, eingesetzt werden.

3. Verfahren nach Anspruch 1 oder 2, dadurch gekennzeichnet, daß ein Hydrophobierungsmittel eingesetzt wird, das aus
70 bis 89 Gew.-% Hexamethyldisiloxan und/oder Trimethylsilanol,
10 bis 30 Gew.-% Hexamethyldisilazan und/oder Divinyltetramethyldisilazan und
1 bis 5 Gew.-% Wasser besteht, wobei sich die Angaben in Gew.-% auf das Gesamtgewicht des Hydrophobierungsmittels beziehen.

## Claims

1. Process for the preparation of hydrophobic particulate silica by reacting a water repellent based on organosilicon compounds with particulate pyrogenically produced or precipitated silica containing Si-OH groups with simultaneous mechanical loading of the reaction mixture, which process is characterized in that 5 to 50 % by weight of the particulate pyrogenically produced or precipitated silica containing Si-OH groups, relative to the total weight of the reaction mixture, comprising particulate silica and water repellent, the water repellent containing 1 to 5% by weight of water, based on the total weight of the water repellent, are used, with the proviso that the reaction mixture contains no linear polyorganosiloxane of the general formula 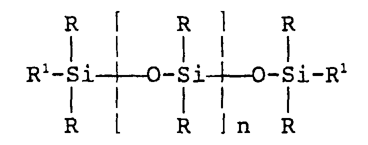 where R represents identical or different monovalent substituted or unsubstituted hydrocarbon radicals, R' has the same meaning as R and can additionally represent an OH radical, and n is a positive integer.

2. Process according to Claim 1, characterized in that 20 to 30 % by weight of the particulate pyrogenically produced or precipitated silica containing Si-OH groups, relative to the total weight of the reaction mixture comprising particulate silica and water repellent, are used.

3. Process according to Claim 1 or 2, characterized in that a water repellent is used, which comprises
70 to 89 % by weight of hexamethyldisiloxane and/or trimethylsilanol,
10 to 30 % by weight of hexamethyldisilazane and/or divinyltetramethyldisilazane and
1 to 5 % by weight of water, where the data in % by weight relate to the total weight of water repellent.

## Revendications

1. Procédé de préparation de dioxyde de silicium particulaire hydrophobe par réaction d'un agent d'hydrophobisation à base de composés organosiliciés contenant des groupes Si-OH, de dioxyde de silicium particulaire pyrogène ou précipité sous contraintes mécaniques simultanées du mélange réactionnel, caractérisé en ce qu'on utilise 5 à 50% en poids de dioxyde de silicium particuliare hydrophobe pyrogène ou précipité, contenant des groupes Si-OH, par rapport au poids total du mélange, composé de dioxyde de silicium particulaire hydrophobe pyrogène ou précipité, l'agent d'hydrophobisation contenant 1 à 5% en poids d'eau par rapport au poids total du mélange, à la condition que le mélange réactionnel ne contienne pas de polyorganosiloxane linéaire de formule générale 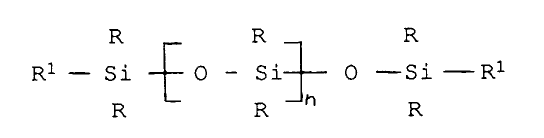 R représentant des radicaux hydrocarbonés identiques ou différents monovalents substitués ou non substitués, R¹ ayant la même signification que R et en outre peut représenter un radical OH et n est un nombre entier positif.

2. Procédé selon la revendication 1, caractérisé en ce qu'on utilise de 20 à 30% en poids de dioxyde de silicium particulaire hydrophobe pyrogène ou précipité, contenant des groupes Si-OH, par rapport au poids total du mélange réactionnel composé de dioxyde de silicium particulaire et d'agent d'hydrophobisation.

3. Procédé selon la revendication 1 ou 2, caractérisé en ce qu'on utilise un agent d'hydrophobisation composé
de 70 à 89% en poids d'hexaméthyle disiloxane et/ou de triméthyl silanol,
de 10 à 30% en poids d'hexaméthyldisilazane et/ou de divinyltétraméthyldisilazane et
de 1 à 5% en poids d'eau, les données en % en poids se rapportant au poids total de l'agent d'hydrophobisation.
